# EUROPEAN PATENT APPLICATION

(11) **EP 1 277 759 A1**
(43) Date of publication of application: **22.01.2003**
(21) Application number: 02021843.4
(22) Date of filing: 13.01.1998
(51) Int. Cl.: C07H 19/16, C07H 19/04, C07H 19/06

(54) **Cytokine related treatments of disease**

(30) Priority: 17.01.1997 US 36094 P; 23.04.1997 US 43974 P; 12.08.1997 US 55487 P
(62) Divisional of application: 98903474.9
(71) Applicant: ICN Pharmaceuticals, Inc., Costa Mesa, CA 92626 (US)
(72) Inventor: Tam, Robert, Costa Mesa, CA 92626 (US); Wang, Guangyi, Irvine, CA 92715 (US); Averett, Devron, Irvine, CA 92653 (US); Ramasamy, Kandasamy, Laguna Hills, CA 92653 (US)
(74) Representative: Adams, Harvey Vaughan John

(57) **Abstract**

Nucleosides and other compounds to selectively modulate Th1 and Th2 responses relative to each other in the treatment of disease. In one aspect of the invention, administration of a nucleoside or other compound reduces the dosage at which a primary drug is administered. In another aspect of the invention, an abnormality reflected in increased response in one group of cytokines is treated by administering a nucleoside or other compound which increases response in another group of cytokines. In yet another aspect ofthe invention, apatient is prophylactically treated by administering a nucleoside or other compound which selectively reduces Th1 activity without significantly reducing Th2 activity. In yet another aspect of the invention, a nucleoside or other compound is administered to a patient at a dose which reduces the patient's GTP pool to a degree that selectively reduces one of the Th 1 or Th2 response without significantly reducing the other response. Controlled release dosage forms are particularly contemplated to achieve that result.

## Description

This application claims priority to **(1)** provisional application ser. no. 60/028586 filed April 23, 1997, **(2)** provisional application ser. no. 60/043,974 filed April 23, 1997, **(3)** provisional application ser. no. 60/055,487 filed August 12, 1997; and **(4)** provisional application 60/036,094 dated January 14, 1997.

### FIELD OF THE INVENTION

The present invention relates to the field of nucleosides.

### BACKGROUND OF THE INVENTION

Mammalian immune systems contain two major classes of lymphocytes: B lymphocytes (B cells), which originate in the bone marrow; and T lymphocytes (T cells) which originate in the thymus. B cells are largely responsible for humoral immunity (i.e., antibody production), while T cells are largely responsible for cell-mediated immunity.

T cells are generally considered to fall into two subclasses, helper T cells and cytotoxic T cells. Helper T cells activate other lymphocytes, including B cells and cytotoxic T cells, and macrophages, by releasing soluble protein mediators called cytokines which are involved in cell-mediated immunity. As used herein, lymphokines are a subset of cytokines.

Helper T cells are also generally considered to fall into two subclasses, Th1 and Th2. Th1 cells (also known as Type 1 cells) produce interleukin 2 (IL-2), tumor necrosis factor (TNFα) and interferon gamma (IFNγ), and are responsible primarily for cell-mediated immunity such as delayed type hypersensitivity and antiviral immunity. In contrast, Th2 cells (also known as Type 2 cells) produce interleukins, IL4, IL-5, IL-6, IL-9, IL-10 and IL-13, and are primarily involved in assisting humoral immune responses such as those seen in response to allergens, e.g. IgE and lgG4 antibody isotype switching (Mosmann, 1989, *Annu Rev Immunol*, 7:145-173).

As used herein, the terms Th1 and Th2 "responses" are meant to include the entire range of effects resulting from induction of Th1 and Th2 lymphocytes, respectively. Among other things, such responses include variation in production of the corresponding cytokines through transcription, translation, secretion and possibly other mechanisms, increased proliferation of the corresponding lymphocytes, and other effects associated with increased production of cytokines, including motility effects.

The priority applications, each of which is incorporated herein by reference, relate to aspects of our recent discoveries involving the effect of various nucleosides (which are defined herein to include derivatives and analogs of native nucleosides) on selectively modulating lymphocyte responses relative to each other. Among other things, we have shown that either of Th1 and Th2 responses can be selectively suppressed while the other is either induced or left relatively unaffected, and either of Th1 or Th2 responses can be selectively induced while the other is either suppressed or left relatively unaffected. We have also discovered the surprising fact that nucleosides effective in selectively modulating Th1 and Th2 responses relative to one another tend to have a bimodal effect. Among other things, nucleosides which tend to generally suppress or induce both Th1 and Th1 activity at a relatively higher dose, tend to selectively modulate Th1 and Th2 relative to each other at relatively lower doses.

The mechanisms by which nucleosides and other compounds selectively modulate Th1 and Th2 responses relative to each other are still unclear. One possibility contemplated by the present inventors is that effective nucleosides alter the pool of guanosine triphosphate (GTP), which in turn affects the rate at which cytokines are produced. In this theory, relatively large variations in available.GTP are sufficient to affect concentrations of both Th1 and Th2 cytokines, while relatively smaller variations in available GTP tend to affect concentrations of Th1 and Th2 cytokines to different extents.

The effects of 2-β-D-ribofuranosylthiazole-4-carboxamide (Tiazofurin), a synthetic C-nucleoside analogue, on GTP levels supports this view. Tumor cells are characterized by high levels of inosine monophosphate dehydrogenase (IMP DH) activity, and it is known that IMP DH is the rate-limiting enzyme of GTP biosynthesis. Weber, G., IMP Dehydrogenase and GTP as Targets in Human Leukemia Treatment, **Adv. Exp. Med. Biol.** 309B:287-292 (1991). Tiazofurin has been shown to selectively block IMP DH activity and deplete guanine nucleotide pools, which in turn forces various tumors into remission. Weber, G., Critical Issues in Chemotherapy with Tiazofurin, **Adv. Enzyme Regul.** 29:75-95 (1989). Typical initial doses of Tiazofurin are about 4,400 mg/m², with consolidation doses of about 1100 to 3300 mg/m². At these levels synthesis of both Th1 and Th2 responses are greatly reduced, thereby essentially shutting down much of the immune system. In one aspect of the present invention it is contemplated that much smaller doses of Tiazofurin, in the range of 1/10th to one-half that set forth above, would be sufficient to specifically suppress either a Th1 response or a Th2 response without greatly reducing the other response.

The effects of 1-b-D-ribofuranosyl-1,2,4-triazole-3-carboxamide (Ribavirin) also supports the present theory. Ribavirin is a potent, broad-spectrum antiviral agent, which has also been shown to inhibit IMP DH. Yamada, Y. et al., Action of the Active Metabolites of Tiazofurin and Ribavirin on Purified IMP Dehydrogenase, **Biochem.** 27:2193-2196 (1988). Ribavirin proceeds under a different mechanism than Tiazofurin in inhibiting IMP DH, however, acting on a different site on the enzyme molecule. Ribavirin is converted to its active metabolite, ribavirin-monophosphate (RMP), which inhibits the enzyme at the IMP-XMP site of IMP DH. As with Tiazofurin, the affinity of Ribavirin's active form to the enzyme is higher than that of the natural metabolite. At relatively high doses, approximately 2200 mg/m² or about 1200 - 1500 mg/day for an adult, Ribavirin reduces IMP DH activity to such an extent that both Th1 and Th2 responses are inhibited. At relatively lower dosages of approximately 600 to 1000 mg/day, Ribavirin promotes a Th1 response and suppresses a Th2 response.

Despite the existence of as-yet undefined mechanisms, we have discovered that enormous potential benefits can be derived from selective modulation of Th1 and Th2 responses relative to each other. We have concluded, for example, that specific modulation of Th1 relative to Th2 can be useful in treating a wide variety of conditions and diseases, ranging from infections, infestations, tumors and hypersensitivities to autoimmune diseases.

These discoveries are especially significant because modern treatment strategies for many of the above-listed diseases have either limited effectiveness, significant side effects, or both. Treatment of autoimmune disease, for example, is frequently limited to palliative measures, removal of toxic antibodies (as in myasthenia gravis), and administration of hazardous drugs including corticosteroids, chloroquine derivatives, and antimetabolic or antitumor drugs, and drugs such as cyclosporines which target immune system cells.

### SUMMARY OF THE INVENTION

This application relates to the use of nucleosides in the relatively low dosage range to selectively modulate Th1 and Th2 responses relative to each other in the treatment of disease. In one aspect of the invention, administration of a nucleoside or other compound reduces the dosage at which a primary drug is administered. In another aspect of the invention, an abnormality reflected in increased response in one group of cytokines is treated by administering a nucleoside or other compound which increases response in another group of cytokines. In yet another aspect of the invention, a patient is prophylactically treated by administering a nucleoside or other compound which selectively reduces Th1 activity without significantly reducing Th2 activity. In yet another aspect of the invention, a nucleoside or other compound is administered to a patient at a dose which reduces the patient's GTP pool to a degree that selectively reduces one of the Th1 or Th2 response without significantly reducing the other response. Controlled release dosage forms are particularly contemplated to achieve that result.

Examples of nucleosides contemplated to be effective in this manner are D- and L-forms of: (a) bicyclic nucleosides corresponding to any of Formulas 1, 1-A through 1-F; and (b) monocyclic nucleosides nucleosides corresponding to any of Formulas 2 through 5.

Examples of primary drugs contemplated to be effective in this manner are anti-viral agents such as Ribavirin, acyclovir, and AZT™; anti-fungal agents such as tolnaftate, Fungizone™, Lotrimin,™ Mycelex,™, Nystatin and Amphoteracin; anti-parasitics such as Mintezol™, Niclocide™, Vermox™, and Flagyl™; bowel agents such as Immodium™, Lomotil™ and Phazyme™; anti-tumor agents such as Adriamycin™, Cytoxan™, Imuran™, Methotrxate, Mithracin™, Tiazofurin™, Taxol™; dermatologic agents such as Aclovate™, Cyclocort™, Denorex™, Florone™, Oxsoralen™, coal tar and salicylic acid; migraine preparations such as ergotamine compounds; steroids and immunosuppresants not listed above, including cyclosporins, Diprosone™, hydrocortisone; Floron™, Lidex™, Topicort and Valisone; and metabolic agents such as insulin.

### DETAILED DESCRIPTION

### Definitions

Where the following terms are used in this specification, they are used as defined below.

The terms "α" and "β" indicate the specific stereochemical configuration of a substituent at an asymmetric carbon atom in a chemical structure as drawn.

The term "abnormality" refers to a condition associated with a disease. Thus, Th1 and/or Th2 responses resulting from an autoimmune disease are considered herein to be abnormalities of the corresponding cytokine(s) even though such cytokine responses may commonly result from the disease.

The term "aryl" refers to a monovalent unsaturated aromatic carbocyclic radical having a single ring (e.g., phenyl) or two condensed rings (e.g., naphthyl), which can optionally be substituted with hydroxyl, lower alky, chloro, and/or cyano.

The term "effective amount" refers to the amount of a compound of formula (I) which will restore immune function to normal levels, or increase immune function above normal levels in order to eliminate infection.

The term "enantiomers" refers to a pair of stereoisomers that are non-superimposable mirror images of each other. A mixture of a pair of enantiomers, in a 1:1 ratio, is a "racemic" mixture.

The term "heterocycle" refers to a monovalent saturated or unsaturated carbocyclic radical having at least one hetero atom, such as N, O or S, within the ring each available position of which can be optionally substituted, independently, with, e.g., hydroxy, oxo, amino, imino, lower alkyl, bromo, chloro and/or cyano. Included within this class of substituents are purines, pyrimidines.

The term "immunomodulators" refers to natural or synthetic products capable of modifying the normal or aberrant immune system through stimulation or suppression.

The term "isomers" refers to different compounds that have the same formula. "Stereoisomers" are isomers that differ only in the way the atoms are arranged in space.

The term "L-configuration" is used throughout the present invention to describe the chemical configuration of the ribofuranosyl moiety of the compounds that is linked to the nucleobases. The L-configuration of the sugar moiety of compounds of the present invention contrasts with the D-configuration of ribose sugar moieties of the naturally occurring nucleosides such as cytidine, adenosine, thymidine, guanosine and uridine.

The term "lower alkyl" refers to methyl, ethyl, n-propyl, isopropyl, n-butyl, t-butyl, I-butyl or n-hexyl. This term is further exemplified to a cyclic, branched or straight chain from one to six carbon atoms.

The term "monocyclic" refers to a monovalent saturated carbocyclic radical having at least one hetero atom, such as O, N, S, Se or P, within the ring, each available position of which can be optionally substituted, independently, with a sugar moiety or any other groups like bromo, chloro and/or cyano, so that the monocyclic ring system eventually aromatized [e.g., Thymidine; 1-(2'-deoxy-?-D-erythro-pentofuranosyl)thymine].

The term "nucleoside" refers to a compound composed of any pentose or modified pentose moiety attached to a specific position of a heterocycle or to the natural position of a purine (9-position) or pyrimidine (1-position) or to the equivalent position in an analog, including especiall both D- and L- forms of nitrogenous bicyclic and monocyclic heterocycles depicted in Figures 1, 1-A through 1-F, and 2 through 5 herein.

The term "C-nucleosides" is used throughout the specification to describe the linkage type that formed between the ribose sugar moiety and the heterocyclic base. In C-nucleosides, the linkage originates from the C-1 position of the ribose sugar moiety and joins the carbon of the heterocyclic base. The linkage that forms in C-nucleosides are carbon to carbon type.

The term "D-nucleosides" refers to nucleoside compounds that have a D-ribose sugar moiety (e.g., Adenosine).

The term "L-nucleosides" refers to nucleoside compounds that have an L-ribose sugar moiety.

The term "N-nucleosides" is used throughout the specification to describe the linkage type that formed between the ribose sugar moiety and the heterocyclic base. In N-nucleosides, the linkage originates from the C-1 position of the ribose sugar moiety and joins the nitrogen of the heterocyclic base. The linkage that forms in N-nucleosides are carbon to nitrogen type.

The term "nucleotide" refers to a phosphate ester substituted on the 5'-position of a nucleoside.

The term "pharmaceutically acceptable salts" refers to any salt derived from inorganic and organic acids or bases.

The term "protecting group" refers to a chemical group that is added to, oxygen or nitrogen atom to prevent its further reaction during the course of derivatization of other moieties in the molecule in which the oxygen or nitrogen is located. A wide variety of oxygen and nitrogen protecting groups are known to those skilled in the art of organic synthesis.

The term "purine" refers to nitrogenous bicyclic heterocycles depicted in Figures 1, 1-A through 1-F herein.

The term "pyrimidine" refers to nitrogenous monocyclic heterocycles depicted in Figures 2 through 5 herein.

The term "tumor" refers broadly to all manner of autonomous morbid growth of tissue which may or may not become malignant, including all manner of neoplasms and cancers.

The terms "treating" or "treatment" of a disease refers to executing a protocol, which may include administering one or more drugs to a patient, in an effort to alleviate signs or symptoms of the disease. Thus, "treating" or "treatment" do not require complete alleviation of signs or symptoms, do not require a cure, and specifically include protocols which have only marginal effect on the patient.

### Combinations and Methods

Contemplated combinations in one aspect of the present invention generally include a primary or "first" drug and another or "second" drug, and contemplated methods involve selecting and combining the first and second drugs in combination therapies. In preferred embodiments a disease is identified which is known to produce an abnormality in at least one cytokine in a patient, the first drug is selected from among those compounds demonstrated to treat the disease at a monotherapeutic dosage, and the second drug, which may be a bimodal nucleoside modulator as herein described, is selected from among those compounds known to exacerbate the very abnormality produced by the disease when administered within a given dosage range. The first drug is then administered at less than the monotherapeutic dosage and the second drug is administered in a dosage outside the dosage range which exacerbates the abnormality. Since the second drug has a bimodal activity with respect to at least some of the cytokines of interest, the combination is still effective to treat the disease, and administration of the second drug allows reduction in the administered dosage of the primary or first drug.

Examples of primary drugs contemplated to be effective in combination with a modulator selected from Figures 1, 1-A through 1-F, and 2-5, are anti-viral agents such as interferon, including but not limited to interferon α and γ, Ribavirin, acyclovir, and AZT™; anti-fungal agents such as tolnaftate, Fungizone™, Lotrimin,™ Mycelex,™, Nystatin and Amphoteracin; anti-parasitics such as Mintezol™, Niclocide™, Vermox™, and Flagyl™; bowel agents such as Immodium™; Lomotil™ and Phazyme™; anti-tumor agents such as interferon α and γ, Adriamycin™, Cytoxan™, Imuran™, Methotrexate, Mithracin™, Tiazofurin™, Taxol™; dermatologic agents such as Aclovate™, Cyclocort™, Denorex™, Florone™, Oxsoralen™, coal tar and salicylic acid; migraine preparations such as ergotamine compounds; steroids and immunosuppresants not listed above, including cyclosporins, Diprosone™, hydrocortisone; Floron™, Lidex™, Topicort and Valisone; and metabolic agents such as insulin, and other drugs which may not nicely fit into the above categories, including cytokines such as IL2, IL4, IL6, IL8, IL10 and IL12. Especially preferred primary drugs are AZT, 3TC, 8-substituted guanosine analogs, 2',3'-dideoxynucleosides, interleukin II, interferons such as IαB-interferons, tucaresol, levamisole, isoprinosine and cyclolignans.

Examples of secondary drugs contemplated to be effective in the invention are D- and L- forms of (a) bicyclic nucleosides corresponding to generic Formula 1 and 1-A through 1-F, and (b) monocyclic nucleosides nucleosides corresponding to Formulas 2 through 5. Other nucleoside and non-nucleoside compounds effective in the invention are readily identified through screening of such compounds *in vitro* for effect on IL-2, TNF-α, IFN-γ, IL-4 and IL-5 as described in PCT/US97/00600.

Formula 1 compounds are purine nucleosides having the structure: 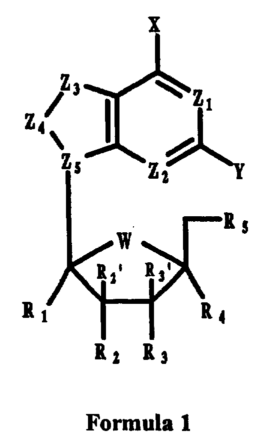 wherein R₁, R₂, R₃, R₄, R₅, R₂' and R₃' are independently selected from the group consisting of H, OH, NH₂, F, Cl, Br, I, N₃, -CN, -OR', -NR'₂, -SR', -NHNH₂, -NHOH, CHO, COOR', CONR'₂, alkyl, alkenyl, alkylnyl, aryl, aralkyl, substituted alkyl, substituted alkenyl, substituted alkynyl, substituted aryl, substituted aralkyl, where the substituent is selected from F, Cl, Br, I, N₃, -CN, -OR", NO₂, -NR"₂, SR", -NHNH₂, -NHOH, COOR", CONR"₂ and where R' and R" are H, alkyl, alkenyl, alkynyl, aryl, aralkyl;
W = O, S, CH₂, Se;
Z₁, Z₂ are independently selected from N, C, CH;
Z₃, Z₄, Z₅ are independently selected from the group consisting of -CR-, -NR-, -O-, -S-, -Se-, -C=O, -C=S, -S=O, -CR=CR-, -CR=N-, -N=N-, where R is selected from the group consisting of H, F, Cl, Br, I, N₃, -CN, -OR', -NR'₂, -SR', -NHNH₂, -NHOH, -NO₂, CHO, COOR', CONH₂, -C(O)-NH₂, -C(S)-NH₂, -C(NH)-NH₂, -C(NOH)-NH₂, =O, =NH, =NOH, =NR, alkyl, alkenyl, alkylnyl, aryl, aralkyl, substituted alkyl, substituted alkenyl, substituted alkynyl, substituted aryl, substituted aralkyl, where the substituent is selected from H, -OH, NH₂, F, Cl, Br, I, N₃, -CN, -COOR", -CONR"₂, -OR", -NR"₂, -SR", -NHNH₂, -NHOH, -NO₂, and R', R" are H, alkyl, alkenyl, alkynyl, aryl, aralkyl, acetyl, acyl, sulfonyl;

The Chemical bond between Z₃ and Z₄ or Z₄ and Z₅ is selected from C-C, C=C, C-N, C=N, N-N, N=N, C-S, N-S;
X and Y are independently selected from the group consisting of H, OH, NH₂, F, Cl, Br, I, N₃, -S-NH₂, -S(O)-NH₂, -S(O2)-NH₂, -CN, -COOR', -CONR'₂, -OR', -NR'₂, -SR', -NHNH₂, -NHOH, alkyl, alkenyl, alkylnyl, aryl, aralkyl, substituted alkyl, substituted alkenyl, substituted alkynyl, substituted aryl, substituted aralkyl, where the substituent is selected from F, Cl, Br, I, N₃, -CN, -OR", NO₂, -NR"₂, SR", -NHNH₂, -NHOH, and R', R" are H, alkyl, alkenyl, alkynyl, aryl, aralkyl.

Formula 1-A compounds are 8-substituted "-or β- L- or D- guanosine analogs having the structure: 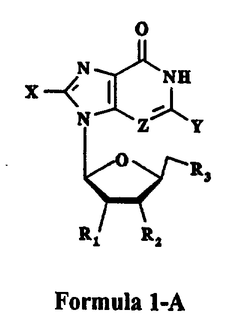 wherein X is selected from H, R, F, Cl, Br, I, N₃, -CN, -OR, -SR, -NR₂, -NHNH₂, -NHOH, -CHO, -CONH₂, -COOR, and -L-A; where R is selected from alkyl, alkenyl, alkynyl, and aralkyl, acetyl, acyl, sulfonyl; L is a linker and selected from alkyl, alkenyl, alkynyl, and aralkyl; and A is selected from H, -OR', -SR', -NR'₂, -NHNR'₂, -CHO, -COOR', -CONR'₂, where R' is selected from H, Me, Et, allyl, acetyl, -COCF₃;
Y is selected from H, R, F, Cl, Br, I, N₃, CN, OR, SR, NR₂, where R is selected from H, alkyl, alkenyl, alkynyl, and aralkyl, acetyl, acyl, sulfonyl;
Z is N or CH; and
R₁, R₂, and R₃ are independently selected from H, -OH, -OAc, -OBz, -OP(O₂)OH.

Formula 1B compounds are 7-substituted-8-oxo-"-or β- L-guanosine analogs having structure:

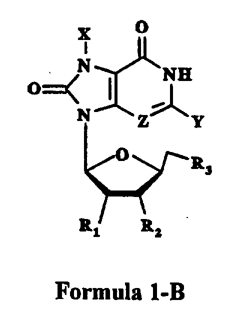

Formula 1-C compounds are 7-deaza-7,8-mono- or disubstituted "-or β- L- or D-guanosine analogs having the structure: 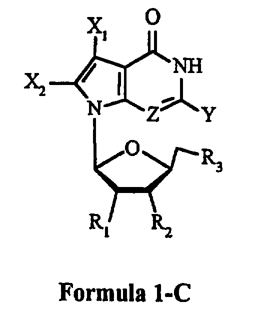 wherein X₁ and X₂ are independently selected from H, R, F, Cl, Br, I, N₃, -CN, -OR, -SR, -NR₂, -NHNH₂, -NHOH, -CHO, -CONH₂, -COOR, and -L-A; where R is selected from alkyl, alkenyl, alkynyl, and aralkyl, acetyl, acyl, sulfonyl; L is a linker and selected from alkyl, alkenyl, alkynyl, and aralkyl; and A is selected from H, -OR', -SR', -NR'₂, -NHNR'₂, -CHO, -COOR', -CONR'₂, where R' is selected from H, Me, Et, allyl, acetyl, -COCF₃;
Y is selected from H, R, F, Cl, Br, I, N₃, -CN, -OR, -SR, -NR₂, where R is selected from H, alkyl, alkenyl, alkynyl, and aralkyl, acetyl, acyl, sulfonyl;
Z is N or CH;
R₁, R₂, and R₃ are independently selected from H, -OH, -OAc, -OBz, -OP(O₂)OH.

Formula 1-D compounds are 7-deaza-8-aza-7-substituted "-or β- L- or D- guanosine analogs having the structure:

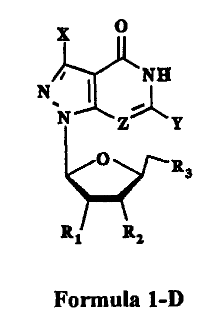

X is selected from H, R, F, Cl, Br, I, N₃, -CN, -OR, -SR, -NR₂, -NHNH₂, -NHOH, -CHO, -CONH₂, -COOR, and -L-A; where R is selected from alkyl, alkenyl, alkynyl, and aralkyl, acetyl, acyl, sulfonyl; L is a linker and selected from alkyl, alkenyl, alkynyl, and aralkyl; and A is selected from H, -OR', SR', -NR'₂, -NHNR'₂, -CHO, -COOR', -CONR'₂, where R' is selected from H, Me, Et, allyl, acetyl, -COCF₃;
Y is selected from H, R, F, Cl, Br, I, N₃, -CN, -OR, -SR, -NR2, where R is selected from H, alkyl, alkenyl, alkynyl, and aralkyl, acetyl, acyl, sulfonyl;
Z is N or CH;
R₁, R₂, and R₃ are independently selected from H, -OH, -OAc, -OBz, -OP(O₂)OH.

Formula 1-E compounds are thiazolo[4,5-d]pyrimidine "-or β- L- or D- nucleosides having the structure: 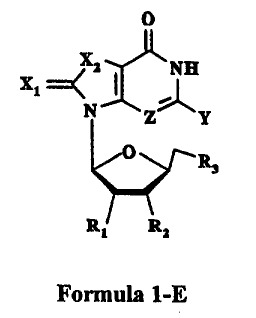 X₁ = O, S, =NH, =NNH₂, =NHOH, =NR where R is selected from alkyl, alkyenyl, alkynyl, and aralkyl, acyl;
X₂ is S, O, or Se
Y is selected from H, R, F, Cl, Br, I, N₃, -CN, -OR, -SR, -NR₂, where R is selected from H, alkyl, alkenyl, alkynyl, and aralkyl, acetyl, acyl, sulfonyl;
Z is N or CH;
R₁, R₂, and R₃ are independently selected from H, -OH, -OAc, -OBz, -OP(O₂)OH.

Formula 1-F compounds are β-L- or D- purine nucleosides having the structure: 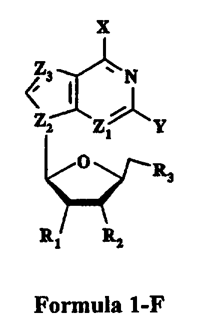 X is selected from H, R, -SNH₂, -S(O)NH₂, -SO₂NH₂, F, Cl, Br, I, N₃, -CN, -OR, -SR, -NR₂, where R is selected from H, alkyl, alkenyl, alkynyl, and aralkyl, acetyl, acyl, sulfonyl;
Y is selected from H, R, F, Cl, Br, I, N₃, -CN, -OR, -SR, -NR₂, where R is selected from H, alkyl, alkenyl, alkynyl, and aralkyl, acetyl, acyl, sulfonyl;
Z₁, Z₂ and Z₃ are independently selected from C, N, and CH;
R₁, R₂, and R₃ are independently selected from H, -OH, -OAc, -OBz, -OP(O₂)OH.

Formula 2 compounds have the structure: 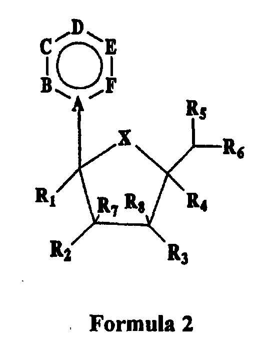 wherein:
A is independently selected from N or C;
B, C, E, F are independently selected from CH, CO, N, S, Se, O, NR¹, CCONH₂, CCH₃, C-R² or P; R¹ is independently H, lower alkyl, lower alkylamines, COCH₃, lower alkyl alkenyl, lower alkyl vinyl or lower alkyl aryls. R² is independently H, OH, halogens, CN, N₃, NH₂, C(=O)NH₂, C(=S)NH₂, C(=NH)NH₂.HCl, C(=NOH)NH₂, C(=NH)OMe, lower alkyl, lower alkylamines, lower alkyl alkenyl, lower alkyl vinyl, lower alkyl aryls or substituted heterocycles;
D is independently selected from CH, CO, N, S, Se, O, NR¹, CCONH₂, CCH₃, C-R², P or nothing, where R¹ is independently H, O, lower alkyl, lower alkylamines, COCH₃, lower alkyl alkenyl, lower alkyl vinyl or lower alkyl aryls, and R² is independently H, OH, halogens, CN, N₃, NH₂, lower alkyl, lower alkylamines, lower alkyl alkenyl, lower alkyl vinyl, lower alkyl aryls or substituted heterocycles;
X is independently O, S, CH₂ or NR; where R is COCH₃;
R₁ and R₄ are independently selected from H, CN, N₃, CH₂OH, lower alkyl and lower alkyl amines;
R₂, R₃, R₅, R₆, R₇ and R₈ are independently selected from H, OH, CN, N₃, halogens, CH₂OH, NH₂, OCH₃, NHCH₃, ONHCH₃, SCH₃, SPh, alkenyl, lower alkyl, lower alkyl amines and substituted heterocycles; and
R₁, R₂, R₃, R₄, R₅, R₆, R₇ and R₈ are not all substituted at the same time; such that
when R₂ = R₃ = H, then R₇ and R₈ are hydrogens or nothing;
when R₁, R₄ or R₅ are substituted, then R₇ = R₈ = H and R₂ = R₃ = OH;
when R₂ or R₃ are substituted, then R₇ and R₈ are H or OH;
when R₇ or R₈ are substituted, then R₂ and R₃ are H or OH;
when R₇ and R₈ are hydroxyl, then R₂ and R₃ are not OH;
when A = N; B = CO; C = N or NH; D = CO or C-NH₂; E is CH or C-substituted; F = CH; X = O, S or CH₂, then R₂ will not be H, OH, CH₃, halogens, N₃, CN, SH, SPh, CH₂OH, CH₂OCH₃, CH₂SH, CH₂F, CH₂N₃, aryl, aryloxy or heterocycles;
when A = N; B = CO; C = N or NH; D = CO or C-NH₂; E is CH, C-CH₃ or halogen; F = CH; X = N-COCH₃, then R₂ will not be H or OH;
when A = N; B = CH; C = CH or CH₃; D = CH or C-CH₃; E is CH, C- CH₃ or C-CONH₂; F = CH; X = O, or CH₂, then R₂ will not be H or OH;
when A = N; B = N, CO or CH; C = CH, C-Cl or C-OCH₃; D = CH or C-Ph; E is CH, C-Cl or C-Ph; F = N or CO; X = O, then R₂ will not be H or OH;
when A = N; B = CO or CS; C = N or NH; D = CO or C-NH₂; E is CH or N; F = N or CH; X = O, then R₂ will not be H or OH; and
when A = C; B = CH; C = NH; D = CO, CS or C-NH₂; E is N or NH; F = CO or CH; X = O, then R₂ will not be H or OH.

Formula 3 compounds have the structure: 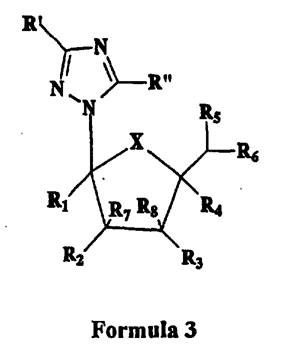 wherein:
X is independently O, S, CH₂ and NR, where R is COCH₃;
R' and R" are independently selected from H, CN, C(=O)NH₂, NH₂, C(=S)NH₂, C(=NH)NH₂.HCl, C(=NOH)NH₂, C(=NH)OMe, heterocycles, halogens, lower alkyl or lower alkyl aryl;
R₁ and R₄ are independently selected from H, CN, N₃, CH₂OH, lower alkyl or lower alkyl amines; and
R₂, R₃, R₅, R₆, R₇ and R₈ are independently selected from H, OH, CN, N₃, halogens, CH₂OH, NH₂, OCH₃, NHCH₃, ONHCH₃, SCH₃, SPh, alkenyl, lower alkyl, lower alkyl amines or substituted heterocycles; such that
when R₂ = R₃ = H, then R₇ and R₈ are hydrogens or nothing.

In compounds of Formula 3, R' is preferably carboxamide or CN and R" is hydrogen or halogens; R₁ = R₄ = R₅ = R₇ = R₈ = H and R₂ = R₃ = OH, and preferably X is oxygen.

Formula 4 compounds have the structure: 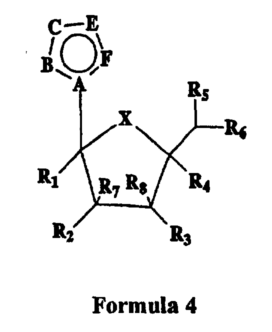 wherein:
A is independently selected from N or C;
B, C, E and F are independently selected from CH, CO, N, S, Se, O, NR¹, CCONH₂, CCH₃, C-R² or P; R¹ is independently H, lower alkyl, lower alkylamines, COCH₃, lower alkyl alkenyl, lower alkyl vinyl or lower alkyl aryls. R² is independently H, OH, halogens, CN, N₃, NH₂, C(=O)NH₂, C(=S)NH₂, C(=NH)NH₂.HCl, C(=NOH)NH₂, C(=NH)OMe, lower alkyl, lower alkylamines, lower alkyl alkenyl, lower alkyl vinyl, lower alkyl aryls or substituted heterocycles;
X is independently O, S, CH₂ or NR; where R is COCH₃;
R₁ and R₄ are independently selected from H, CN, N₃, CH₂OH, lower alkyl or lower alkyl amines; and
R₂, R₃, R₅, R₆, R₇ and R₈ are independently selected from H, OH, CN, N₃, halogens, NH₂, CH₂OH, OCH₃, NHCH₃, ONHCH₃, SCH₃, SPh, alkenyl, allyl, lower alkyl, lower alkyl amines or substituted heterocycles; such that
when R₂ = R₃ = H, then R₇ and R₈ are hydrogens or nothing;
when A is carbon; B = E = N; C is N-Ph, then F is not CH;
when A = N; C is CH; B = E = C-CH₃, then F is not nitrogen; and
when A is carbon, B = N; C = C-CONH₂; E = CH; F = S, then X is not CH₂.

In compounds of Formula 4, R¹ is preferably H, lower alkyl or allyl; R² is preferably H, OH, halogens, CN, N₃, NH₂, C(=O)NH₂, C(=S)NH₂, C(=NH)NH₂.HCl, C(=NOH)NH₂ or C(=NH)OMe; and when R₁ = R₄ = R₅ = R₇ = R₈ = H, then preferably R₂ = R₃ = OH and preferably X is oxygen.

Formula 5 compounds have the structure: 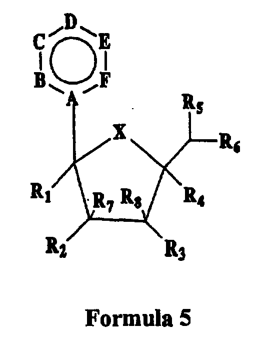 wherein:
A is independently selected from N or C;
B, C, E, F are independently selected from CH, CO, N, S, Se, O, NR¹, CCONH₂, CCH₃, C-R² or P; R¹ is independently H, lower alkyl, lower alkylamines, COCH₃, lower alkyl alkenyl, lower alkyl vinyl or lower alkyl aryls. R² is independently H, OH, halogens, CN, N₃, NH₂, C(=O)NH₂, C(=S)NH₂, C(=NH)NH₂.HCl, C(=NOH)NH₂, C(=NH)OMe, lower alkyl, lower alkylamines, lower alkyl alkenyl, lower alkyl vinyl, lower alkyl aryls or substituted heterocycles;
D is independently selected from CH, CO, N, S, Se, O, NR¹, CCONH₂, CCH₃, C-R², P or nothing; R¹ is independently H, O, lower alkyl, lower alkylamines, COCH₃, lower alkyl alkenyl, lower alkyl vinyl or lower alkyl aryls. R² is independently H, OH, halogens, CN, N₃, NH₂, lower alkyl, lower alkylamines, lower alkyl alkenyl, lower alkyl vinyl, lower alkyl aryls or substituted heterocycles;
X is independently O, S, CH₂ or NR where R is COCH₃;
R₁ and R₄ are independently selected from H, CN, N₃, CH₂OH, lower alkyl and lower alkyl amines; and
R₂, R₃, R₅, R₆, R₇ and R₈ are independently selected from H, OH, CN, N₃, halogens, CH₂OH, NH₂, OCH₃, NHCH₃, ONHCH₃, SCH₃, SPh, alkenyl, lower alkyl, lower alkyl amines and substituted heterocycles; such that
when R₂ = R₃ = H, then R₇ and R₈ are hydrogens or nothing.
when A = N; B = CO; C = N or NH; D = CO or C-NH₂; E is CH or C-substituted; F = CH; X = O, S or CH₂, then R₂ will not be H, OH, CH₃, halogens, N₃, CN, SH, SPh, CH₂OH, CH₂OCH₃, CH₂SH, CH₂F, CH₂N₃, aryl, aryloxy or heterocycles.
when A = N; B = CO; C = N or NH; D = CO or C-NH₂; E is CH, C-CH₃ or halogen; F = CH; X = N-COCH₃, then R₂ will not be H or OH;
when A = N; B = CH; C = CH or CH₃; D = CH or C-CH₃; E is CH, C- CH₃ or C-CONH₂; F = CH; X = O, or CH₂, then R₂ will not be H or OH;
when A = N; B = N, CO or CH; C = CH, C-Cl or C-OCH₃; D = CH or C-Ph; E is CH, C-Cl or C-Ph; F = N or CO; X = O, then R₂ will not be H or OH;
when A = N; B = CO or CS; C = N or NH; D = CO or C-NH₂; E is CH or N; F = N or CH; X = O, then R₂ will not be H or OH; and
when A = C; B = CH; C = NH; D = CO, CS or C-NH₂; E is N or NH; F = CO or CH; X = O, then R₂ will not be H or OH.

In another aspect of the invention, an abnormality reflected in increased response in one group of cytokines is treated by administering a nucleoside or other compound which increases response in another group of cytokines. Thus, for example, a common rapid onset type of allergy results in an abnormally elevated Th2 response. The abnormality is treated by administering Ribavirin at between 600 mg/day and 1,000 mg/day (for a typical adult), at which dose the Th1 response is induced. The treatment is effective because Th1 and Th2 have a teeter-totter type relationship in this instance, such that the Th2 response is suppressed.

In yet another aspect of the invention, a patient is prophylactically treated by administering a nucleoside or other compound which selectively reduces Th1 activity without significantly reducing Th2 activity. The prophylaxis can, for example, prepare the patient for organ or tissue transplant, or for anticipated contact with allergens.

In yet another aspect of the invention, a nucleoside or other compound is administered to a patient at a dose which reduces the patient's GTP pool to a degree that selectively reduces one of the Th1 or Th2 response without significantly reducing the other response. Controlled release dosage forms are particularly contemplated to achieve that result, especially formulations which maintain the dose of the compound in the serum within a desirable range. In the case of Ribavirin, for example, the serum level should be maintained between about 2 µM and about 5 µM. In terms of delivery rates, a controlled release formulation may advantageously have an *in vitro* dissolution rate when measured by the USP Paddle Method at 100 rpm at 900 ml aqueous buffer (pH between 1.6 and 7.2) between about 15% and 40% by weight of the compound after 1 hour, between about 30% and about 50% by weight of the compound after 2 hours, about 50% and 70% by weight of the compound after 4 hours, between about 60% and about 80% by weight of the compound after 6 hours

### Uses

It is contemplated that the claimed combinations will be used to treat a wide variety of conditions, and in fact any condition which responds positively to administration of one or more such combinations. Among other things it is specifically contemplated that such combinations may be used to treat an infection, an infestation, a tumor, a hypersensitivity or an autoimmune disease.

Infections contemplated to be treated with the compounds of the present invention include respiratory syncytial virus (RSV), hepatitis B virus (HBV), hepatitis C virus (HCV), herpes simplex type 1 and 2, herpes genitalis, herpes keratitis, herpes encephalitis, herpes zoster, human immunodeficiency virus (HIV), influenza A virus, hantann virus (hemorrhagic fever), human papilloma virus (HPV), measles and fungus. It is especially contemplated that combinations claimed herein will be useful in treating chronic viral and bacterial infections, including HIV, Tuberculosis, leprosy and so forth.

Infestations contemplated to be treated with the compounds of the present invention include intracellular protozoan infestations, as well as helminth and other parasitic infestations. Again, it is especially contemplated that combinations claimed herein will be useful in treating chronic infestations.

Tumors contemplated to be treated include those caused by a virus, and the effect may involve inhibiting the transformation of virus-infected cells to a neoplastic state, inhibiting the spread of viruses from transformed cells to other normal cells and/or arresting the growth of virus-transformed cells.

Hypersensitivities contemplated to be treated include all types of allergies, including IgE and IgG allergies, hyper IgE syndrome, and dermatic conditions such as atopic dermatitis. It is also contemplated that claimed combinations can be used to treat transplant rejection, (graft vs. host disease) and implant reactions.

Autoimmune diseases can be classified as either non-organ-specific or organ-specific. Non-organ-specific autoimmune diseases include rheumatoid arthritis, gout and gouty arthritis, Systemic Lupus Erythematosus (SLE), Sjogren syndrome, scleroderma, polymyositis and dermomyositis, ankylosing spondylitis, and rheumatic fever. Organ-specific autoimmune diseases are known for virtually every organ, including insulin-dependent diabetes, thyroid diseases (Graves disease and Hashimoto thyroiditis), Addison disease, and some kidney and lung diseases including allergy and asthma, multiple sclerosis, myasthenia gravis, uveitis, psoriasis, forms of hepatitis and cirrhosis, celiac disease, inflammatory bowel disease, and some types of male and female infertility. Autoimmune processes may also be stimulated by viral infections including the HIV virus, may result from rejection of transplantation, and may accompany certain tumors, or be precipitated by exposure to some chemicals.

It is also contemplated that an abnormality reflected in increased response in one group of cytokines can be treated by administering a nucleoside which increases response in another group of cytokines. Thus, for example, since common IgE allergies are associated with a predominantly Th2 response, allergies can be treated with Ribavirin, which increases Th1 response at low dosages of about 500 mg/day to about 1,000 mg/day.

In yet another aspect of the invention, a patient is prophylactically treated by administering a compound which selectively reduces Th1 activity without significantly reducing Th2 activity. The prophylactic treatment may be to reduce expected undesirable effects from an upcoming event, such as an organ or tissue transplant, or to reduce symptoms from an expected pulmonary insult, as from the onset of increase in airborne pollen levels in Spring.

### Synthesis

Synthesis of compounds according to Formulas 1, and 1-A through 1-F were set forth in co-pending PCT application PCT/US97/18387, which is incorporated herein as though set forth in full. Synthesis of compounds according to Formulas 2 through 5 were set forth in co-pending PCT application PCT/US97/00600.

### Administration

It is contemplated that compounds according to the present invention will be administered in any appropriate pharmaceutical formulation, and under any appropriate protocol. Where primary or "first" treatment drugs are employed as discussed above, preferred monotherapeutic dosages and protocols for such drugs are set forth in the PDR, or are at least available from the manufacturer or distributor. Preferred dosages and protocols for the "second" drugs, such as bi-modal nucleosides described herein, may best be established through experimentation with particular patients. Such experimentation need not be extensive, and it is contemplated that "second" drugs comprising nucleosides as described herein will be administered at between about 100 mg/day and about 5,000 mg/day.

Of course, one of ordinary skill in the art will recognize that a therapeutically effective amount will vary with the infection or condition to be treated, its severity, the treatment regimen to be employed, the pharmacokinetics of the agent used, as well as the patient (animal or human) treated. Thus, effective dosages may range from 1 mg/kg of body weight, or less, to 25 mg/kg of body weight or more. In general a therapeutically effective amount of the "second" drug is contemplated to range from slightly less than about 1 mg./kg. to about 25 mg./kg. of the patient, depending upon the compound used, the condition or infection treated and the route of administration. This dosage range generally produces effective blood level concentrations of active compound ranging from about 0.04 to about 100 micrograms/cc of blood in the patient. It is contemplated, however, that appropriate patient-specific regimens will be developed by administering a small amount, and then increasing the amount until either the side effects become unduly adverse, or the intended effect is achieved.

Administration of compounds according to the present invention may take place orally, parenterally (including subcutaneous injections, intravenous, intramuscularly, by intrasternal injection or infusion techniques), by inhalation spray, or rectally, topically and so forth, and in dosage unit formulations containing conventional non-toxic pharmaceutically acceptable carriers, adjuvants and vehicles.

It is contemplated that compounds according to the present invention can be formulated in admixture with a pharmaceutically acceptable carrier. For example, the compounds of the present invention can be administered orally as pharmacologically acceptable salts. Because the compounds of the present invention are mostly water soluble, they can be administered intravenously in physiological saline solution (e.g., buffered to a pH of about 7.2 to 7.5). Conventional buffers such as phosphates, bicarbonates or citrates can be used for this purpose. Of course, one of ordinary skill in the art may modify the formulations within the teachings of the specification to provide numerous formulations for a particular route of administration without rendering the compositions of the present invention unstable or compromising their therapeutic activity. In particular, the modification of the present compounds to render them more soluble in water or other vehicle, for example, may be easily accomplished by minor modifications (salt formulation, esterification, *etc.*) which are well within the ordinary skill in the art. It is also well within the ordinary skill of the art to modify the route of administration and dosage regimen of a particular compound in order to manage the pharmacokinetics of the present compounds for maximum beneficial effect in patients.

In certain pharmaceutical dosage forms, the pro-drug form of administered compounds, especially including acylated (acetylated or other) derivatives, pyridine esters and various salt forms of the present compounds are preferred. One of ordinary skill in the art will recognize how to readily modify the present compounds to pro-drug forms to facilitate delivery of active compounds to a target site within the host organism or patient. One of ordinary skill in the art will also take advantage of favorable pharmacokinetic parameters of the pro-drug forms, where applicable, in delivering the present compounds to a targeted site within the host organism or patient to maximize the intended effect of the compound.

In addition, compounds included in combinations according to the present invention may be administered separately or together, and when administered separately this may occur in any order. The amounts of the active ingredient(s) and pharmaceutically active agent(s) and the relative timings of administration will be selected in order to achieve the desired combined therapeutic effect.

Administration routes of compounds according to the present invention may range from continuous (intravenous drip) to several oral administrations per day (for example, Q.I.D.) and may include oral, topical, parenteral, intramuscular, intravenous, sub-cutaneous, transdermal (which may include a penetration enhancement agent), buccal and suppository administration, among other routes of administration.

To prepare therapies according to the present invention, a therapeutically effective amount of a compound is preferably intimately admixed with a pharmaceutically acceptable carrier according to conventional pharmaceutical compounding techniques to produce a dose. A carrier may take a wide variety of forms depending on the form of preparation desired for administration, e.g., oral or parenteral. In preparing pharmaceutical compositions in oral dosage form, any of the usual pharmaceutical media may be used. Thus, for liquid oral preparations such as suspensions, elixirs and solutions, suitable carriers and additives including water, glycols, oils, alcohols, flavoring agents, preservatives, coloring agents and the like may be used. For solid oral preparations such as powders, tablets, capsules, and for solid preparations such as suppositories, suitable carriers and additives including starches, sugar carrier, such as dextrose, mannitol, lactose and related carriers, diluents, granulating agents, lubricants, binders, disintegrating agents and the like may be used. If desired, the tablets or capsules may be enteric-coated or sustained release by standard techniques.

For parenteral formulations, the carrier will usually comprise sterile water or aqueous sodium chloride solution, though other ingredients including those which aid dispersion may be included. Of course, where sterile water is to be used and maintained as sterile, the compositions and carriers must also be sterilized. Injectable suspensions may also be prepared, in which case appropriate liquid carriers, suspending agents and the like may be employed.

It will also be appreciated that in general, the most preferred uses according to the present invention are those in which the active compounds are relatively less cytotoxic to the non-target host cells and relatively more active against the target. In this respect, it may also be advantageous that L-nucleosides may have increased stability over D-nucleosides, which could lead to better pharmacokinetics. This result may attain because L-nucleosides may not be recognized by enzymes, and therefore may have longer half-lives.

Thus, therapies have been disclosed which employ nucleosides and other compounds to selectively modulate Th1 and Th2 responses relative to each other in the treatment of disease. While specific embodiments have been disclosed herein, the scope of the invention is not be limited except through interpretation of the appended claims.

### Preferred aspects of the invention

One preferred aspect of the present invention provides

A method of reducing an administered dosage of a first drug in the treatment of a disease which is known to produce an abnormality in at least one cytokine in a patient, comprising:
identifying a monotherapeutic dosage of the first drug which is effective to treat the disease;
identifying a second drug which is known to exacerbate the abnormality when administered within a dosage range; and
administering a combination therapy comprising the first drug at less than the monotherapeutic dosage and the second drug outside the dosage range.

In certain embodiments of this aspect, the disease comprises a chronic disease,
the disease comprises chronic viral disease,
the disease comprises insulin dependent diabetes,
the disease comprises allergy,
the disease comprises atopic dermatitis,
the disease comprises intracellular protozoan infection,
the disease comprises hyper IgE syndrome,
the disease comprises HIV,
the disease comprises graft versus host disease,
the disease comprises Systemic Lupus Erythematosus, or
the disease comprises a tumor.

Optionally in this aspect, in particular in these embodiments, the abnormality comprises an abnormal increase in Th1 activity,
the abnormality comprises an abnormal decrease in Th1 activity,
the abnormality comprises an abnormal increase in Th2 activity,
the abnormality comprises an abnormal decrease in Th2 activity,
the second drug comprises a pharmaceutically acceptable form of a nucleoside,
the second drug comprises a pharmaceutically acceptable form of a D-nucleoside,
the second drug comprises a pharmaceutically acceptable form of an L-nucleoside,
the second drug comprises a pharmaceutically acceptable form of Ribavirin,
the second drug comprises a pharmaceutically acceptable form of an interferon,
the second drug comprises a nucleoside according to at least one of Formulas 1, 1A, 1B, 1C, 1D, 1E or 1F, or
the second drug comprises a nucleoside according to at least one of Formulas 2, 3, 4 or 5.

In a second preferred aspect, the invention provides:
A method of treating a disease in a patient, comprising:
   associating the disease with an increase in activity of a first lymphokine phenotype;
   associating administration of a pharmaceutical within a first dosage range with an increase in activity of a second cytokine phenotype;
   administering the pharmaceutical within the first dosage range to treat the disease.

In certain embodiments of this second preferred aspect, the disease comprises a chronic disease,
the disease comprises chronic viral disease,
the disease comprises insulin dependent diabetes mellitus,
the disease comprises allergy,
the disease comprises atopic dermatitis,
the disease comprises intracellular protozoan infections
the disease comprises hyper IgE syndrome,
the disease comprises HIV,
the disease comprises graft versus host disease,
the disease comprises Systemic Lupus Erythematosus, or
the disease comprises a tumor.

In this second aspect, and in particular in these embodiments, the method may further comprise associating decreased activity of the second cytokine phenotype with administration of the pharmaceutical within a second dosage range,
in which case, the pharmaceutical may comprise a phannaceutically acceptable form of Ribavirin or
a pharmaceutically acceptable form of an interferon.

Likewise in this aspect, and in particular in those embodiments, the method may further comprise
identifying a therapeutic agent which is recognized as being effective by itself to treat the disease at a recommended dosage; and
administering the therapeutic agent at less than recommended dosage in combination with the pharmaceutical,
in which case: the therapeutic agent may be selected from the list consisting of anti-viral agents, anti-fungal agents, bowel agents, anti-tumor agents, dermatologic agents, migraine preparations, steroids, immuno-suppresants and metabolic agents,
and if it is so selected, the method may further comprise associating decreased activity of the second cytokine phenotype with administration of the pharmaceutical within a second dosage range; or
the pharmaceutical comprises a pharmaceutically acceptable form of Ribavirin or an Interferon; or
the method further comprises associating decreased activity of the second cytokine phenotype with administration of the pharmaceutical within a second dosage range.

Once again in this aspect, and in particular in the above-mentioned embodiments, the second drug may comprise a nucleoside according to at least one of Formulas 1, 1A, 1B, 1C, 1D, 1E or 1F, or at least one of Formulas 2, 3, 4 or 5, in particular where the method comprises identifying a therapeutic agent which is recognized as being effective by itself and administering said agent at less than the recommended dosage, as described above.

In a third preferred aspect, the invention provides:
A method of prophylactically treating a patient comprising:
   providing a pharmaceutical which suppresses Th1 activity in the patient when administered below a given dosage level; and
   administering the pharmaceutical to the patient below the given dosage level.

In certain embodiments of this aspect, the prophylaxis comprises preparing the patient for an organ transplant, or
the prophylaxis comprises preparing the patient for a tissue transplant.

Optionally in this aspect, in particular in these embodiments,
the method further comprises providing the pharmaceutical at a dosage which induces Th2 activity, or
the pharmaceutical comprises a nucleoside according to at least one of Formulas 1, 1A, 1B, 1C, 1D, 1E or 1F, or
the pharmaceutical comprises a nucleoside according to at least one of Formulas 2, 3, 4 or 5.

In other embodiments ofthis third aspect, the prophylaxis comprises preparingthe patient for expected contact with allergens.

In a fourth preferred aspect, the invention provides:
A method of treating a disease having an elevated or suppressed Th1 or Th1 response, comprising:
   identifying a compound and a dosage range for the compound which reduces the patient's GTP pool to a degree that selectively reduces one of the Th1 or Th2 response without significantly reducing the other response; and
   administering the nucleoside to the patient within the dosage range.

In certain embodiments of this aspect, the disease comprises a chronic disease,
the disease comprises chronic viral disease,
the disease comprises insulin dependent diabetes mellitus,
the disease comprises allergy,
the disease comprises atopic dermatitis,
the disease comprises intracellular protozoan infection,
the disease comprises hyper IgE syndrome,
the disease comprises HIV,
the disease comprises graft versus host disease,
the disease comprises Systemic Lupus Erythematosus, or
the disease comprises a tumor.

Optionally in this aspect, in particular in these embodiments, the compound comprises a nucleoside according to at least one of Formulas 1, 1A, 1B, 1C, 1D, 1E or 1F, or the compound comprises a nucleoside according to at least one of Formulas 2, 3, 4 or 5.

In a fifth aspect the invention provides
A controlled release preparation for oral administration including a compound effective to selectively modulate Th1 and Th2 responses with respect to each other within a dosage range.

In certain embodiments of this aspect, the compound comprises a nucleoside according to at least one of Formulas 1, 1A, 1B, 1C, 1D, 1E or 1F,
the compound comprises a nucleoside according to at least one of Formulas 2, 3, 4 or 5,
the compound comprises Ribavirin, or
the compound comprises an interferon.

Optionally in this aspect, in particular in these embodiments, the patient maintains a serum level of between about 2 µM and about 5 µM of the compound, or
the preparation has an *in vitro* dissolution rate when measured by the USP Paddle Method at 100 rpm at 900 ml aqueous buffer (pH between 1.6 and 7.2) between about 15% and 40% by weight of the compound after 1 hour, between about 30% and about 50% by weight of the compound after 2 hours, about 50% and 70% by weight of the compound after 4 hours, between about 60% and about 80% by weight of the compound after 6 hours.

## Claims

1. A nucleoside having the structure

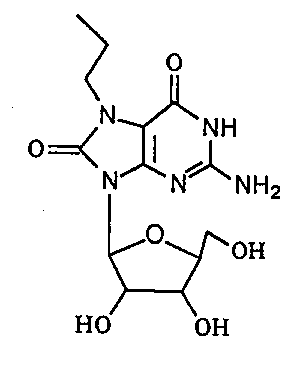

2. A nucleoside having the structure
